# EUROPÄISCHE PATENTSCHRIFT

(11) **EP 4 017 427 B1**
(45) Veröffentlichungstag und Bekanntmachung des Hinweises auf die Patenterteilung: **11.12.2024**
(21) Anmeldenummer: 20758160.4
(22) Anmeldetag: 17.08.2020
(51) Int. Cl.: A61F 2/60, A61F 2/74, A61F 2/64, A61F 2/66

(54) **ORTHOPÄDIETECHNISCHE EINRICHTUNG UND ENERGIESPEICHEREINRICHTUNG**
ORTHOPAEDIC DEVICE AND ENERGY STORAGE DEVICE
DISPOSITIF ORTHOPÉDIQUE ET DISPOSITIF DE STOCKAGE D'ÉNERGIE

(30) Priorität: 20.08.2019 DE 102019122372
(43) Veröffentlichungstag der Anmeldung: 29.06.2022
(73) Patentinhaber: Ottobock SE & Co. KGaA, 37115 Duderstadt (DE)
(72) Erfinder: WILL, Christian, 37079 Göttingen (DE); PARTH, Torsten, 63329 Engelsbach (DE); VIER, Leonard, 37176 Nörten-Hardenberg (DE); MOENICKE, Carsten, 37115 Duderstadt (DE); GEHRMANN, Georg, 37085 Göttingen (DE); BOHLAND, Andreas, 1070 Wien (AT); BELTRAN ULLAURI, Jessica, Gabriela, 37412 Herzberg (DE); HÖRIG, Viktor, Gerhard, 37421 Herzberg (DE); BOITEN, Herman, 6715LE Ede (NL)
(74) Vertreter: Gramm, Lins & Partner Patent- und Rechtsanwälte PartGmbB
(86) Internationale Anmeldenummer: PCT/EP2020/072968
(87) Internationale Veröffentlichungsnummer: WO 2021/032663

(56) Entgegenhaltungen:
- WO-A1-2018/161023
- GB-A- 2 343 848

## Beschreibung

Die Erfindung betrifft eine Energiespeichereinrichtung nach Anspruch 1.

Orthopädietechnische Einrichtungen sind aus dem Stand der Technik in vielen Formen bekannt. Dazu zählen beispielsweise Prothesen, insbesondere Knieprothesen, Knöchelprothesen, Fußprothesen, Ellenbogenprothesen oder Handprothesen. Zu orthopädietechnischen Einrichtungen zählen auch Orthesen, insbesondere Knieorthesen, Knöchelorthese, Fußorthesen, Ellenbogenorthesen oder Handorthesen. Aus der WO 2018/161023 A1 ist beispielsweise ein Prothesenkniegelenk bekannt, das ein Hydrauliksystem mit einem Ausgleichsbehälter aufweist.

Auch Exoskelette, die außen an einem Körperteil oder dem gesamten Körper des Trägers angeordnet werden und vom Körper selbst nicht mehr durchzuführende Bewegungen und/oder Tätigkeiten ermöglichen sollen, sind orthopädietechnische Einrichtungen im Sinne dieser Erfindung. Dazu zählen auch Vorrichtungen, die es dem Träger erleichtern, kraftaufwendige, anstrengende oder ermüdende Tätigkeiten, beispielsweise Überkopfarbeiten, besser, leichter, schneller und länger auszuführen.

Viele dieser orthopädietechnischen Einrichtungen verfügen über Gelenke, durch die nicht mehr vorhandene Gelenke des Trägers der orthopädietechnischen Einrichtung ersetzt oder unterstützt, gestützt oder geschützt werden sollen. In vielen Fällen ist es gewünscht und vorteilhaft, wenn die orthopädietechnische Einrichtung bei einer Bewegung beispielsweise eines ihrer Gelenke, Energie aufnimmt, diese zwischenspeichert und zu einem späteren Zeitpunkt, beispielsweise in einem Schrittzyklus, wieder abgibt. Dies ist beispielsweise bei Knieprothesen und Knieorthesen gewünscht, bei denen vorzugsweise beim Einbeugen des Knies Energie in einer Energiespeichereinrichtung gespeichert wird, die zum Strecken des Knies wieder freigegeben wird.

Aus dem Stand der Technik ist eine Reihe von Energiespeichereinrichtungen bekannt, die in der Regel als Federelemente, beispielsweise hydraulische oder mechanische Federelemente, ausgebildet sind. Wird das Gelenk gebeugt, wird das Federelement komprimiert mit Energie aufgeladen. Diese wird vom Federelement zu einem späteren Zeitpunkt wieder abgegeben. Nachteilig ist jedoch, dass diese Energieabgabe unmittelbar nach dem Wegfall der für die Energieaufladung verantwortlichen Kraft erfolgt und unkontrolliert und instantan verläuft. Eine Energiezwischenspeicherung oder eine kontrolliertere Abgabe der gespeicherten Energie ist mit derart einfachen Systemen nicht oder kaum möglich.

Aus dem Stand der Technik sind daher Dämpfungselemente oder Widerstände bekannt, durch die eine Bewegung beispielsweise des Gelenkes der orthopädietechnischen Einrichtung erschwert oder verzögert wird. Dies ist beispielsweise bei hydraulischen Systemen der Fall, bei denen beispielsweise zwischen zwei Zylinderkammern als Arbeitsmedium ein Fluid bewegt wird, wenn der Kolben der hydraulischen Einrichtung bewegt wird. Befindet sich in der Fluidleitung zwischen den beiden Zylinderkammern ein Drosselventil, kann mit diesem Drosselventil der Strömungswiderstand, der dem strömenden Fluid entgegengesetzt wird, eingestellt werden. Wird das Ventil vollständig geschlossen, kann kein Fluid strömen und das Gelenk der orthopädietechnischen Einrichtung ist blockiert.

Nachteilig ist jedoch, dass mit derartigen Systemen die Energie nicht gespeichert werden kann, sodass lediglich ein passiver Widerstand erreicht wird.

Will man beide Effekte, also die Energiespeicherung und die kontrollierte Abgabe, gleichzeitig erreichen, werden in der Regel beide Systemarten miteinander kombiniert. So sind beispielsweise aus der US 9,416,838 B2 und der WO 2016/171548 entsprechende Systeme bekannt. Da diese jedoch eigentlich Kombinationen zweier Systeme sind, sind die Energiespeichereinrichtungen konstruktiv aufwendig und damit fehleranfällig und kostenintensiv.

Zudem ist es bei all diesen Systemen kaum möglich, eine freie Bewegung des Gelenkes der orthopädietechnischen Einrichtung zu ermöglichen.

Der Erfindung liegt daher die Aufgabe zugrunde, eine Energiespeichereinrichtung so weiter zu entwickeln, dass sie flexibel einsetzbar und platzsparender sowie konstruktiv einfacher hergestellt werden kann.

Die Erfindung löst die gestellte Aufgabe durch eine Energiespeichereinrichtung gemäß dem Oberbegriff des Anspruchs 1, die sich dadurch auszeichnet, dass die Energiespeichereinrichtung wenigstens ein Ausgleichsvolumen, das mit der Fluidleitung durch eine Fluidverbindung fluidtechnisch verbunden ist, und wenigstens ein erstes steuerbares Ventil aufweist, durch das die Fluidverbindung geöffnet und geschlossen werden kann.

Ist die Fluidverbindung durch das erste steuerbare Ventil geschlossen, kann kein Fluidaustausch zwischen dem Ausgleichsvolumen und den übrigen Bestandteilen, insbesondere der ersten Zylinderkammer oder der zweiten Zylinderkammer, stattfinden. Wird in diesem Zustand der Kolben bewegt, wird das Arbeitsmedium aus einer der beiden Zylinderkammern durch die Fluidleitung in die jeweils andere Zylinderkammer gedrückt. Dabei wirkt dem Arbeitsmedium der Strömungswiderstand, der durch die Fluidleitung hervorgerufen wird, entgegen, sodass eine je nach Größe des Strömungswiderstandes stärkere oder weniger starke Dämpfung hervorgerufen wird.

In einer bevorzugten Ausgestaltung verändert sich beim Verschieben des Kolbens innerhalb des Zylinders bei geschlossenem ersten steuerbaren Ventil das für das Arbeitsmedium, also die Flüssigkeit, insgesamt zur Verfügung stehende Volumen.

Herkömmlicherweise ist beispielsweise der Kolben an einer Kolbenstange befestigt und wird an dieser Stange innerhalb des Zylinders verschoben. Dies bedeutet, dass das Volumen der Zylinderkammer, in der sich die Kolbstange befindet, durch die Kolbenstange reduziert wird. Verschiebt sich folglich der Kolben so, dass ein größerer Abschnitt der Kolbenstange in der jeweiligen Zylinderkammer angeordnet wird, wird das insgesamt für das Arbeitsmedium zur Verfügung stehende Volumen verringert, sodass der Druck erhöht wird. Je nach Kompressibilität des Arbeitsmediums kann auf diese Weise eine Bewegung zumindest nahezu, bevorzugt sogar vollständig unterbunden werden, beispielsweise wenn das Fluid, das das Arbeitsmedium bildet, inkompressibel ist. Ist das Arbeitsmedium nicht vollständig inkompressibel, dann wird der dem Verschieben des Kolbens entgegenstehende Widerstand mit zunehmender Verschiebung verstärkt. Das Arbeitsmedium innerhalb der Zylinderkammern und der Fluidleitung wird auf diese Weise komprimiert und dient so als Energiespeicher. Al vollständig inkompressibel wird im Rahmen der vorliegenden Erfindung ein Arbeitsmedium vorzugsweise dann angesehen, wenn die bei der bestimmungsgemäßen Verwendung der orthopädietechnischen Einrichtung die auftretenden Kräfte nicht zu einer Verschiebung des Kolbens bei geschlossener Fluidverbindung führen.

Die Energiespeichereinrichtung dient in dieser Ausgestaltung bei geschlossenem ersten steuerbaren Ventil als Federelement. Die Federkonstante hängt dabei maßgeblich von dem Kompressionsmodul des verwendeten Arbeitsmediums ab. Wird das System belastet, also beispielsweise der Kolben mit einer Kraft beaufschlagt, die in Richtung auf die erste Zylinderkammer wirkt, kommt es zu einer Verschiebung des Kolbens in diese Richtung. Dadurch wird in diesem Ausführungsbeispiel der Anteil der Kolbenstange, der innerhalb des Zylinders angeordnet ist, vergrößert, so dass sich wie oben bereits dargelegt das für das Arbeitsmedium zur Verfügung stehende Volumen verringert. Dadurch wird der Druck auf das Arbeitsmedium erhöht und die Verschiebung des Kolbens endet, wenn der Druck des Arbeitsmediums die von außen auf den Kolben aufgebrachte Kraft kompensiert.

Wie groß bei gegebener äußerer Kraft der Federweg, also die Verschiebung des Kolbens ist, hängt daher davon ab, wie groß die Volumenänderung des Volumens im Verhältnis zum Gesamtvolumen, das dem Arbeitsmedium zur Verfügung steht, ist, das dem Arbeitsmedium zur Verfügung steht. Diese Volumenänderung hängt von dem Durchmesser der Kolbenstange ab, die zu der Volumenveränderung führt. Je kleiner der Durchmesser der Kolbenstange ist, desto kleiner ist die Härte der Feder und desto größer ist der Federweg. Um eine weiche Feder zu erhalten ist es daher von Vorteil, eine besonders dünne, also mit kleinem Durchmesser ausgestattete Kolbenstange zu verwenden. Dies geht jedoch unterhalb eines kritischen Durchmessers zu Lasten der mechanischen Stabilität der Kolbenstange. Die Federkonstante hängt bevorzugt zudem von dem Volumen der ersten Zylinderkammer und dem Volumen der zweiten Zylinderkammer ab. Die Federkonstante kann auch als vom Verhältnis der beiden Volumina abhängig formuliert werden. Es lässt sich folglich bei gegebenem Durchmesser der Kolbenstange die Federkonstante verringern, also der Federweg bei gegebener Kraft vergrößern, indem das Gesamtvolumen, das dem Arbeitsmedium zur Verfügung steht, vergrößert wird. Dadurch wird die Energiespeichereinrichtung in der Regel jedoch vergrößert und benötigt daher mehr Bauraum.

In einer bevorzugten Ausgestaltung erstreckt sich daher die Kolbenstange in, besonders bevorzugt durch, beide Zylinderkammern, verfügt jedoch in den beiden Zylinderkammern über verschiedene Durchmesser oder Querschnittsflächen. Zur Volumenänderung trägt dann nur die Differenz bei, so dass auch kleine Volumenänderungen und damit weiche Federn realisiert werden können.

Eine Kolbenstange hat beispielsweise einen Durchmesser von weniger als 10 mm besonders bevorzugt weniger als 7 mm.

Vorzugsweise ist eine Wand des Zylinders so ausgebildet, dass sie als mechanischer Energiespeicher dient. Dies kann beispielsweise durch einen Bereich mit sehr geringer Wandstärke erreichet werden, der sich bei Einwirkung entsprechender Drücke elastisch verformt.

Die Speicherung von Energie kann verhindert werden, indem das erste steuerbare Ventil verwendet wird, um die Fluidverbindung zwischen dem Ausgleichsvolumen und den übrigen Bestandteilen des Hydrauliksystems zu öffnen. In diesem Fall verändert sich beim Verschieben des Kolbens innerhalb des Zylinders zwar weiterhin das Gesamtvolumen der beiden Zylinderkammern, dies kann jedoch durch das Ausgleichsvolumen ausgeglichen werden, sodass keine Komprimierung des Arbeitsmediums stattfindet und daher auch keine Energie gespeichert wird. Eine Dämpfung der Bewegung des Kolbens findet weiterhin statt, da noch immer der Strömungswiderstand der Fluidleitung dem Transport des Arbeitsmediums entgegensteht. Verkleinert sich beim Verschieben des Kolbens innerhalb des Zylinders das innerhalb der beiden Zylinderkammern insgesamt zur Verfügung stehende Volumen, wird ein Teil des Arbeitsmediums durch die Fluidleitung und die Fluidverbindung in das Ausgleichsvolumen gedrückt. Bei der entgegengesetzten Bewegung des Kolbens innerhalb des Zylinders wird das Arbeitsmedium wieder aus dem Ausgleichsvolumen herausgesaugt, sodass der ursprüngliche Zustand wieder hergestellt wird. Auf diese Weise lässt sich die Energiespeichereinrichtung als dämpfender Energiespeicher verwenden oder sie ermöglicht eine Bewegung des Kolbens, ohne dass Energie zwischengespeichert wird.

Ist das erste steuerbare Ventil geöffnet und das Ausgleichsvolumen folglich nicht vom Rest des Systems getrennt, kann auch ein Temperaturausgleich erfolgen. Dabei wird eine Volumenänderung des Arbeitsmediums aufgrund von Temperaturänderungen durch das Ausgleichsvolumen kompensiert. Dadurch kann verhindert werden, dass sich die Federeigenschaften mit der Temperatur ändern.

In einer bevorzugten Ausgestaltung der Erfindung ist das Arbeitsmedium eine kompressible Flüssigkeit, bevorzugt ein Öl, besonders bevorzugt ein Silikonöl ist. Vorteilhaft ist zudem, dass die gespeicherte Energie, die beim Komprimieren der Flüssigkeit gespeichert wird, bei der Entlastung nahezu vollständig wieder abgegeben wird und das Öl durch seine Fluidität, wodurch es formausfüllend eingesetzt werden kann, sehr platzsparend eingesetzt werden kann und hohe Kräfte aufnimmt.

Durch ein derartiges Arbeitsmedium verschwimmen die Grenzen zwischen einem Hydrauliksystem, bei dem eine Flüssigkeit als Arbeitsmedium verwendet wird, die im allgemeinen als inkompressibel angenommen wird, und einem Pneumatiksystem, bei dem als Arbeitsmedium ein Gas verwendet wird. Dieses ist in der Regel kompressibel.

Bei orthopädietechnischen Einrichtungen aus dem Stand der Technik wird in den Hydraulikanordnungen die Kompressibilität der Flüssigkeiten nicht genutzt. Vielmehr werden in der Regel Arbeitsmedien genutzt, die als inkompressibel angesehen werden und auch als solche verwendet werden. Typischerweise sind in solchen Hydrauliken steuerbare Ventile zwischen den beiden Zylinderkammern vorgesehen, um eine Dämpfung der Bewegung zu erreichen und zu steuern. Zusätzlich kann ein dauerhaft zugeschaltetes Ausgleichsvolumen vorhanden sein.

Insbesondere bei der Verwendung von Kniegelenken entstehen durch die auftretenden Kräfte hohe Drücke von bis zu 200 bar, die auf das Arbeitsmedium der Hydraulikanordnung des Kniegelenkes wirken. Bei typischen Volumina von 25 ml Hydrauliköl und einem Kolbendurchmesser von 25 mm und einem Kolbenstangendurchmesser von 10 mm bedeutet dies, dass sich bei herkömmlichem Hydrauliköl und geschlossenen Ventilen lediglich ein maximaler Kolbenweg von ca. 0,4 mm ergibt und damit zu wenig, um einen spürbaren Federeffekt zu erhalten bzw. um relevante Energiemenge über einen nutzbaren Kolbenweg zu speichern. Ein solches Hydrauliköl gilt im Sinne der vorliegenden Erfindung in dieser Hydraulikanordnung als vollständig inkompressibel. Ein Sperren der Ventile entspricht daher in diesem Fall einem vollständig gesperrten Gelenk ohne Energiespeicherfunktion. Ein Öffnen der Ventile entspricht daher in diesem Fall einem vollständig freien Gelenk ohne Energiespeicherfunktion. Über die Ventilstellung ist eine Dämpfung regelbar, ein steuerbares Ventil vor dem Ausgleichsvolumen ist somit weder notwendig noch vorhanden.

Bei der erfindungsgemäßen Einrichtung wird dagegen die Kompressibilität der Flüssigkeit zur Energiespeicherung genutzt. Dazu wird das Verhältnis zwischen Kompressionsmodul der Flüssigkeit, Kolbenstangendurchmesser und Volumen der Zylinderkammern so gewählt, dass die gewünschten Energien gespeichert werden können, ohne dass zu hohe Drücke entstehen oder der Einfahrweg des Kolbens zu gering wird.

Eine beispielhafte Anforderung an ein Kniegelenk ist es, genügend Energie zu speichern, um den Anwender beim Aufstehen zu unterstützen. Das Ausgleichsvolumen muss nun über ein steuerbares Ventil verfügen um zwischen der Energiespeicherfunktion und der Dämpfungsfunktion umschalten zu können. Bei zugeschaltetem Ausgleichsvolumen verhält sich die Hydraulik wie oben beschrieben, Sperrung und Dämpfung sind über wenigstens ein steuerbares Ventil in der Fluidleitung möglich. Bei geschlossener Fluidverbindung ist das Ausgleichsvolumen vom Rest des Systems fluidtechnisch abgekoppelt. Werden gegebenenfalls vorhandene Ventile in der Fluidleitung geöffnet oder ist ein solches Ventil nicht vorhanden, kann Energie im System gespeichert werden, eine freie Bewegung ist dagegen nicht mehr möglich.

Vorzugsweise hat die als Arbeitsmedium verwendete Flüssigkeit ein Kompressionsmodul von weniger als 1,5 GPa, besonders bevorzugt weniger als 1,2 GPa. Dadurch ist es möglich, die übrigen Parameter, also insbesondere Druck, Volumen der jeweiligen Zylinderkammern und Kolbenstangendurchmesser, in einem technisch einfacher zu realisierenden Bereich zu wählen und insbesondere die Einrichtung kleiner zu bauen, etwa in für orthopädietechnische Einrichtungen vertretbaren Größenordnungen. Bei dem oben bespielhaft genannten Kniegelenk mit herkömmlicher Hydraulik und 25 ml Arbeitsmedium lässt sich dadurch ein längerer Kolbenweg bei dem gleichen maximalen Druck von 200 bar beispielsweise erreichen, indem an Stelle des herkömmlichen Hydrauliköles Silikonöl mit einem Kompressionsmodul von 1,5 GPa verwendet wird und der Durchmesser der Kolbenstange auf 6 mm reduziert wird. Hierdurch ergibt sich bei geschlossener Fluidverbindung, also abgekoppeltem Ausgleichsvolumen und geöffneten zweiten steuerbaren Ventilen, ein Kolbenweg von 7 mm bis die maximalen 200 bar. Bei Verwendung eines Arbeitsmediums mit einem Kompressionsmodul von 1 GPa sind sogar 10.5 mm erreichbar.

Durch die Wahl von Kolbenstangendurchmesser, Volumen der Zylinderkammern und Kompressionsmodul können unterschiedliche Federkonstanten, also Steifigkeiten eingestellt werden. Bevorzugt wird in der Standphase des Gangzyklus eine natürliche Kniesteifigkeit abgebildet, was einer linearen Federkonstante in einem Bereich zwischen 0 N/mm bis 750 N/mm entspricht. Bevorzugt wird eine Federkonstante kleiner als 600 N/mm, besonders bevorzugt kleiner als 400 N/mm, und vorzugsweise größer als 100 N/mm, besonders bevorzugt größer als 300 N/mm eingestellt. Bei einer beispielhaften Ausprägung der Federkonstante von 400 N/mm wirkt unter einem Beugewinkel von 25° eine Kraft von 4320 N. In diesem Zustand ist eine potentielle Energie von etwa 23 Joule gespeichert. Weiterhin wird bei der einwirkenden Kraft ein Weg von 10,8 mm bei vollständig abgekoppeltem Ausgleichsvolumen erreicht.

Vorzugsweise ist das Arbeitsmedium eine magneto-rheologische Flüssigkeit. Diese Flüssigkeiten haben eine Viskosität oder Fließfähigkeit, die sich durch die Einwirkung magnetischer Felder beeinflussen lässt. In diesen Ausgestaltungen können Drosselventile und/oder steuerbare Ventile als Magneten, beispielsweise Elektromagneten, ausgestaltet sein. Dadurch werden teure und aufwändige mechanische Passteile, wie sie für herkömmliche mechanische Ventile benötigt werden, überflüssig. Eine Leitung, durch die das Arbeitsmedium fließt, beispielsweise die Fluidleitung, wird so angeordnet, dass ein Magnetfeld eines Magneten auf sie einwirken kann. Wird das Magnetfeld erhöht, verringert sich beispielsweise die Fließfähigkeit des als magneto-rheologische Flüssigkeit ausgebildeten Arbeitsmediums. Dadurch wird der Strömungswiderstand erhöht. Umgekehrt wird der Strömungswiderstand gesenkt, indem das Magnetfeld abgeschwächt wird, da sich dadurch die Fließfähigkeit eines derartigen Arbeitsmediums erhöht.

Vorzugsweise befindet sich in der Fluidleitung, die die beiden Zylinderkammern miteinander verbindet, wenigstens ein zweites steuerbares Ventil, durch das ein Strömungswiderstand der fluidtechnischen Verbindung vorzugsweise stufenlos einstellbar ist. Dabei ist die fluidtechnische Verbindung vorzugsweise durch das zweite steuerbare Ventil vollständig verschließbar. Vorzugsweise handelt es sich bei dem zweiten steuerbaren Ventil um ein Drosselventil. Alternativ dazu ist ein Drosselventil zusätzlich vorhanden, um den Strömungswiderstand einzustellen. So kann eine Dämpfung der Bewegung des Kolbens innerhalb des Zylinders eingestellt werden.

Besonders bevorzugt befinden sich in der Fluidleitung wenigstens zwei zweite steuerbare Ventile, vorzugsweise zwei Drosselventile, zwischen denen sich die Verbindung zur Fluidverbindung befindet. Auf diese Weise ist sichergestellt, dass das Arbeitsmedium, also das Fluid, immer durch eines der Drosselventile geleitet wird, wenn es in das oder aus dem Ausgleichsvolumen geleitet wird.

Durch die beiden zweiten steuerbaren Ventile, vorzugsweise die beiden Drosselventile, können für die beiden Bewegungsrichtungen des Kolbens innerhalb des Zylinders unterschiedliche Strömungswiderstände und damit unterschiedliche Dämpfungseigenschaften eingestellt werden.

Bevorzugt wird wenigstens eines, besonders bevorzugt jedes der Drosselventile, von einem Rückschlagventil überbrückt, das einen Fluss des Arbeitsmediums in die jeweilige Zylinderkammer erlaubt, aus dieser Zylinderkammer heraus jedoch unterbindet.

In einer bevorzugten Ausgestaltung verfügt die Energiespeichereinrichtung über wenigstens ein Zusatzvolumen, das mit der ersten Zylinderkammer fluidtechnisch in Verbindung steht, wobei die Energiespeichereinrichtung vorzugsweise ein drittes steuerbares Ventil aufweist, durch das die Verbindung geöffnet und geschlossen werden kann. Besonders bevorzugt steht das Zusatzvolumen fluidtechnisch in Verbindung mit der wenigstens einen Fluidleitung, die die beiden Zylinderkammern miteinander verbindet.

Durch dieses Zusatzvolumen werden weitere Möglichkeiten eröffnet, die Energiespeichereinrichtung zu verwenden.

Ein Ausgleichsvolumen ist in der Lage, Arbeitsmedium aufzunehmen, ohne dass dabei der Druck auf das Arbeitsmedium erhöht wird. Damit kann, wie bereits dargelegt, die Volumenänderung der beiden Zylinderkammern, die beim Verschieben des Kolbens entstehen kann, ausgeglichen werden. Es findet folglich ein Druckausgleich statt. Im Gegensatz dazu ist bei einem Zusatzvolumen ein solcher Druckausgleich nicht möglich. Es handelt sich folglich um ein geschlossenes, vorzugsweise vollständig mit Arbeitsmedium gefülltes Volumen. Bei einer Energiespeichereinrichtung, die über wenigstens eines dieser Zusatzvolumina verfügt, wird folglich beim Verschieben des Kolbens innerhalb des Zylinders auch der Druck des Mediums im Zusatzvolumen erhöht oder verringert, wenn das dritte steuerbare Ventil, das die Verbindung zum Ausgleichsvolumen steuert, geschlossen ist. Wird beispielsweise der Kolben innerhalb des Zylinders so verschoben, dass das für das Arbeitsmedium zur Verfügung stehende Volumen der beiden Zylinderkammern verkleinert wird, und ist gleichzeitig das erste steuerbare Ventil, das die Fluidverbindung öffnen und schließen kann, in der geschlossenen Position, so erhöht sich der Druck innerhalb des Arbeitsmediums nicht nur innerhalb der Zylinderkammern, sondern auch innerhalb des Zusatzvolumens.

In diesem Zustand kann das dritte steuerbare Ventil, das für die Verbindung des Zusatzvolumens zu dem Hydrauliksystem verantwortlich ist, geschlossen werden, sodass innerhalb des Zusatzvolumens das Arbeitsmedium unter erhöhtem Druck, und damit mit mehr potentieller Energie, gespeichert wird. Das Zusatzvolumen dient folglich als abschließbarer Energiespeicher, in dem einmal aufgenommene Energie durch das Schließen des betreffenden dritten steuerbaren Ventils gespeichert werden kann. Unabhängig von der Position und/oder der Bewegung des Kolbens innerhalb des Zylinders kann zu jedem gewünschten Zeitpunkt das entsprechende dritte steuerbare Ventil wieder geöffnet werden, um das Arbeitsmedium innerhalb des Zusatzvolumens zu entspannen und die darin gespeicherte, potentielle Energie abzugeben.

Im Falle eines Kniegelenkes kann die Energie beispielsweise beim Hinsetzen gespeichert werden. Anschließend kann das dritte steuerbare Ventil geschlossen und das wenigstens eine zweite steuerbare Ventil in der Fluidleitung und das erste steuerbare Ventil und damit die Fluidverbindung geöffnet werden. Dadurch geht die in den Zylinderkammern gespeicherte Energie verloren, dafür lässt sich jedoch das Gelenk im Sitzen frei bewegen. Beim Aufstehen kann dann das wenigstens eine erste steuerbare Ventil in der Fluidverbindung wieder geschlossen und das dritte steuerbare Ventil geöffnet werden, sodass die im Zusatzvolumen gespeicherte Energie als Aufstehunterstützung genutzt werden kann.

Weiterhin kann über das Zusatzvolumen eine Steifigkeitsänderung durch Öffnen oder Schließen des dritten steuerbaren Ventils erfolgen. Wird das dritte steuerbare Ventil geöffnet, steht insgesamt ein größeres Volumen an Arbeitsmedium zur Verfügung, wodurch sich die Steifigkeit verringert. Durch Schließen des dritten steuerbaren Ventils wird das Gesamtvolumen, das dem Arbeitsmedium zur Verfügung steht, verringert und die Steifigkeit erhöht. Dies kann zur situationsabhängigen Anpassung der Steifigkeit genutzt werden, so ist bei einem Kniegelenk beispielsweise eine größere Steifigkeit in der Standphase erforderlich als beispielsweise beim Hinsetzen. Weiterhin kann auch eine Steifigkeitsanpassung an den Nutzer der orthopädietechnischen Einrichtung sinnvoll sein, beispielsweise abhängig vom Gewicht des Nutzers und seinen persönlichen Vorlieben.

Besonders bevorzugt befindet sich auch in dieser Verbindung ein Drosselventil, sodass der Strömungswiderstand der Verbindung eingestellt werden kann. Dieses Drosselventil kann entweder zusätzlich zu dem dritten steuerbaren Ventil vorhanden sein oder das dritte steuerbare Ventil ist als Drosselventil ausgebildet. Auf diese Weise kann zudem eingestellt werden, wie schnell das unter Druck gelagerte Arbeitsmedium entspannt und über welchen Zeitraum und mit welcher Geschwindigkeit die darin gespeicherte, potentielle Energie abgegeben wird.

In einer bevorzugten Ausgestaltung verfügt die Energiespeichereinrichtung über mehrere Zusatzvolumina. Diese sind vorzugsweise alle mit dem Rest des Fluidsystems, beispielsweise mit einer der Zylinderkammern, verbunden. Besonders bevorzugt verfügt die Energiespeichereinrichtung zudem über eine Mehrzahl von dritten steuerbaren Ventilen, sodass die Verbindungen der einzelnen Zusatzvolumina, bevorzugt jedes einzelnen Zusatzvolumens, separat geöffnet und geschlossen werden kann. Dies geschieht bevorzugt unabhängig voneinander. Auf diese Weise können unterschiedliche Mengen an potentieller Energie in unterschiedlichen Zusatzvolumina gespeichert und bei Bedarf freigegeben werden. Die Zusatzvolumina können das gleiche Volumen oder unterschiedliche Volumina aufweisen und unterschiedlich druckbeständige Behälter sein. Mehrere Zusatzvolumina ermöglichen darüber hinaus eine größere Bandbreite an einstellbaren Steifigkeiten.

Vorzugsweise sind mehrere dieser Zusatzvolumina seriell fluidtechnisch miteinander verbunden. Dies bedeutet, dass die Volumina "in Serie geschaltet" sind. Der Teil des Arbeitsmediums, der in das letzte dieser Zusatzvolumina hineingeleitet wird, muss folglich zunächst durch alle anderen Zusatzvolumina, die mit diesem letzten Zusatzvolumen in Serie geschaltet sind, hindurch.

Alternativ oder zusätzlich dazu sind mehrere der Zusatzvolumina parallel fluidtechnisch miteinander verbunden. Dies bedeutet, dass die einzelnen Volumina "parallel geschaltet" sind. Damit ist nichts über die räumliche Orientierung der Volumina gesagt. Es bedeutet lediglich, dass ein Arbeitsmedium, das in eines der Zusatzvolumina geleitet werden soll, nicht durch ein anderes dieser parallel geschalteten Zusatzvolumina hindurchgeleitet werden muss.

Auch hier ist bevorzugt jedes einzelne Zusatzvolumen mit dem Rest des Fluidsystems durch ein drittes steuerbares Ventil verbindbar oder von diesem trennbar. Besonders bevorzugt ist für jedes Zusatzvolumen ein separates Drosselventil vorhanden, durch das die Strömungswiderstände in den jeweiligen Verbindungsleitungen einstellbar sind.

Vorteilhafterweise verfügt die orthopädietechnische Einrichtung über wenigstens eine elektrische Steuerung, die eingerichtet ist, die steuerbaren Ventile, die Schaltventile und/oder die Drosselventile unabhängig voneinander anzusteuern. Eine derartige elektrische Steuerung ist beispielsweise eine elektronische Datenverarbeitungseinrichtung, die eingerichtet ist, Steuersignale an die entsprechenden Ventile zu senden und so die Ventile von einem Zustand in einen anderen Zustand zu bringen. Dies kann beispielsweise auf der Basis von Sensordaten geschehen, die Sensoren ermitteln, die ebenfalls Teil der orthopädietechnischen Vorrichtung sein können. Dies können Kraftsensoren, Dehnungssensoren, Temperatursensoren, Geschwindigkeits- oder Beschleunigungssensoren oder andere Sensoren sein.

Vorzugsweise ist der erste Kolben entlang einer Kreisbahn verschieblich gelagert, wie dies beispielsweise von Rotationshydrauliken bekannt ist.

Vorteilhafterweise handelt es sich bei der orthopädietechnischen Einrichtung um eine Knieprothese oder eine Knieorthese.

Die Erfindung löst die gestellte Aufgabe zudem durch eine Energiespeichereinrichtung für eine der hier beschriebenen orthopädietechnischen Einrichtungen.

Mit Hilfe der beigefügten Zeichnungen werden nachfolgend einige Ausführungsbeispiele der vorliegenden Erfindung näher erläutert.

Es zeigen:
- Figuren 1 bis 5: - verschiedene Zustände einer Energiespeichereinrichtung gemäß einem ersten Ausführungsbeispiel der vorliegenden Erfindung und
- Figuren 6 bis 10: - verschiedene Zustände einer zweiten Ausführungsform einer Energiespeichereinrichtung.

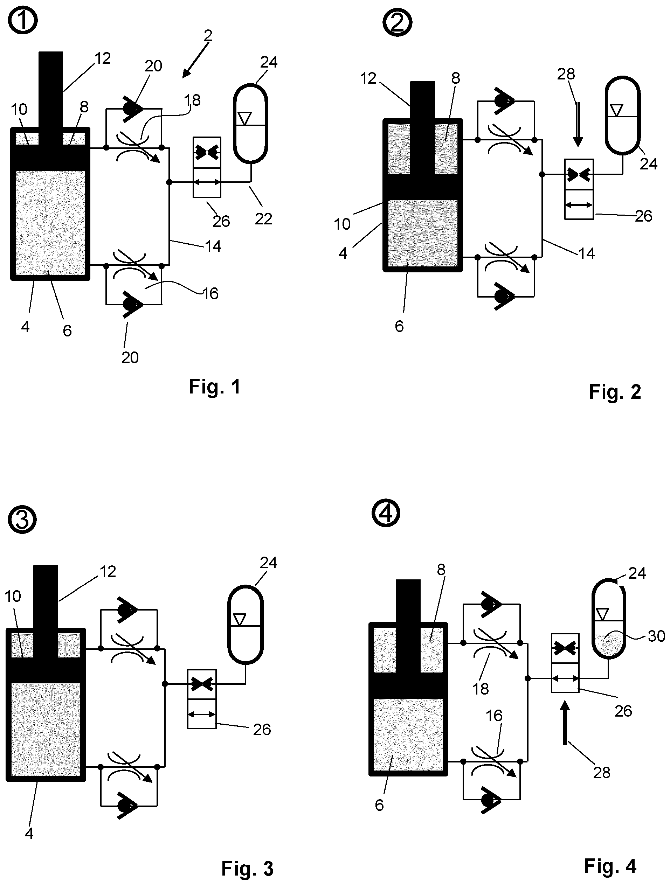

Figur 1 zeigt schematisch eine Energiespeichereinrichtung 2 für eine orthopädietechnische Einrichtung. Die Energiespeichereinrichtung verfügt über einen Zylinder 4, in dem sich eine erste Zylinderkammer 6 und eine zweite Zylinderkammer 8 befinden, die von einem Kolben 10, der in einer Kolbenstange 12 gelagert ist, getrennt werden.

Über eine Fluidleitung 14 ist die erste Zylinderkammer 6 mit der zweiten Zylinderkammer 8 verbunden. In der Fluidleitung 14 befindet sich ein erstes Drosselventil 16 und ein zweites Drosselventil 18, die jeweils von einem Rückschlagventil 20 überbrückt werden. Die Rückschlagventile 20 sind so angeordnet, dass bei geschlossenem ersten Drosselventil 16 kein Arbeitsmedium aus der ersten Zylinderkammer 6 und bei geschlossenem zweiten Drosselventil 18 kein Arbeitsmedium aus der zweiten Zylinderkammer 8 entweichen kann. Im gezeigten Ausführungsbeispiel bilden das erste Drosselventil 16 mit dem ihm zugeordneten Rückschlagventil 20 gemeinsam ein zweites steuerbares Ventil. Das zweite Drosselventil 18 und das ihm zugeordnete Rückschlagventil 20 bilden ebenfalls ein zweites steuerbares Ventil.

Zwischen den beiden Drosselventilen 16, 18 ist über eine Fluidverbindung 22 ein Ausgleichsvolumen 24 mit der Fluidleitung 14 und damit mit der ersten Zylinderkammer 6 und der zweiten Zylinderkammer 8 fluidtechnisch verbunden. In der Fluidverbindung 22 befindet sich ein erstes steuerbares Ventil 26, das in einen geöffneten Zustand, der in Figur 1 dargestellt ist, und in einen geschlossenen Zustand bringbar ist, indem das Ausgleichsvolumen 24 vom Rest des Fluidsystems getrennt wird.

Eine derartige Energiespeichereinrichtung 2, wie sie in den Figuren 1 bis 5 schematisch dargestellt ist, kann beispielsweise in einem Prothesenknie angeordnet werden, sodass ein Schrittzyklus, wie in den Figuren 1 bis 5 beschrieben, ablaufen kann.

Figur 1 zeigt die Situation beim Fersenauftritt. Das Ausgleichsvolumen 24 ist über das geöffnete Schaltventil 26 mit der Fluidleitung 14 verbunden. Das erste Drosselventil 16 und das zweite Drosselventil 18 sind geöffnet, wobei durch die jeweiligen Drosselventile 16, 18 unterschiedliche große Öffnungen erreicht werden können, sodass der einer Fluidbewegung entgegengesetzte Strömungswiderstand einstellbar ist.

Beim Fersenauftritt kommt es zu einer Flexion des Prothesenknies, in das die Energiespeichereinrichtung 2 eingebaut ist. Dadurch wird der Kolben 10 innerhalb des Zylinders 4 nach unten verschoben. Diese Situation ist in Figur 2 dargestellt. Der Kolben 10 ist nach unten verschoben worden, sodass die erste Zylinderkammer 6 verkleinert wurde. Gleichzeitig wurde die zweite Zylinderkammer 8 vergrößert. Das Gesamtvolumen der beiden Zylinderkammern 6, 8 hat jedoch abgenommen, da ein größerer Teil der Kolbenstange 12 nun innerhalb des Zylinders 4 angeordnet ist. Beim Absenken des Kolbens 10 herrschte die in Figur 1 gezeigte Situation vor, sodass das Ausgleichsvolumen 24 mit dem Rest des Fluidsystems verbunden ist. Da sich das Volumen der beiden Zylinderkammern 6, 8 insgesamt beim Absenken des Kolbens 10 verkleinert hat, wurde ein Teil des Fluides ins Ausgleichsvolumen 24 gedrückt.

In Figur 2 ist durch den Pfeil 28 dargestellt, dass beispielsweise beim so genannten "foot flat", bei dem der gesamte Fuß auf dem Boden aufliegt, das erste steuerbare Ventil 26 geschlossen wird. Dadurch wird die Verbindung zum Ausgleichsvolumen 24 und der Fluidleitung 14 getrennt. Der Teil des Arbeitsmedium, der beim Absenken des Fußes und damit beim Absenken des Kolbens 10 innerhalb des Zylinders 4 in das Ausgleichsvolumen 24 gedrückt wurde, kann nun dieses Ausgleichsvolumen 24 nicht mehr verlassen. Eine weitere Flexion des Prothesenknies, in das die Energiespeichereinrichtung 2 eingebaut ist, würde zu einer weiteren Absenkung des Kolbens 10 und damit zu einer weiteren Reduzierung des Gesamtvolumens der beiden Zylinderkammern 6, 8 führen. Dies hätte eine Kompression des enthaltenen Fluids, beispielsweise eines Silikonöls, zur Folge. Dadurch wird der Druck innerhalb des Silikonöls erhöht und somit potentielle Energie gespeichert. Da es für das Arbeitsmedium keine Möglichkeit gibt, das Systems aus erster Zylinderkammer 6, zweiter Zylinderkammer 8 und Fluidleitung 14 zu verlassen, wird die Energie in diesem System gespeichert und beim Nachlassen der flektierenden Kraft wieder abgegeben. Auf diese Weise können beispielsweise bei einem Prothesenknie nun die natürlichen Standphasenflexionswinkel von bis zu 25° erreicht werden, ohne dass der Anwender die Befürchtung haben muss, die gespeicherte Energie sei verloren, sodass er aus dieser Flexion nicht mehr eigenständig das Knie extendieren kann.

Die Energiespeichereinrichtung 2 speichert ab dem Moment des Schließens des Schaltventils 26 die weiter zugeführte potentielle Energie und gibt sie danach wieder ab. Dadurch wird der Kolben 10 in Figur 2 nach oben gedrückt, da der Druck in den beiden Zylinderkammern 6, 8 zwar identisch ist, die dem Druck ausgesetzte Unterseite des Kolbens 10 jedoch größer ist als die dem Druck ausgesetzte Oberseite, sodass insgesamt eine nach oben wirkende Kraft erreicht wird.

Diese Situation ist in Figur 3 dargestellt. Der Kolben 10 mit der Kolbenstange 12 ist nach oben gedrückt worden. Dies geschieht bis zu der Position, an der das erste steuerbare Ventil 26 geschlossen wurde. Geschieht dies, anders als in Figur 2 dargestellt, bereits zu einem früheren Zeitpunkt, also bei einem noch nicht so weit in den Zylinder 4 eingeschobenen Kolben 10 mit Kolbenstange 12, kann die in Figur 3 gezeigt Position erreicht werden. Das Schaltventil 26 bleibt geschlossen.

Wird hingegen, anders als in den gezeigten Figuren, das Schaltventil 26 direkt beim Fersenauftritt geschlossen, also in der in Figur 1 gezeigten Position, befindet sich innerhalb des Ausgleichsvolumens 24 kein Fluid, da bereits vor dem ersten Komprimieren des Volumens der beiden Zylinderkammern 6, 8, das Schaltventil 26 geschlossen wurde.

Durch die gezeigte Anordnung wird es möglich, ab dem Moment, in dem das Schaltventil 26 geschlossen wird, durch das Beugen des Prothesenknies oder eines anderen Gelenkes einer orthopädietechnischen Einrichtung aufgenommene potentielle Energie wieder abzugeben und so dem Träger der orthopädietechnischen Einrichtung bei der umgekehrten Bewegung des Gelenks der orthopädietechnischen Einrichtung zu unterstützen. Bei diesem Vorgang bleibt der Füllstand des Ausgleichsvolumens 24 unverändert.

Figur 4 zeigt die Situation, bei der das erste steuerbare Ventil 26 dem Pfeil 28 entsprechend geöffnet ist. Dies kann bei einem Prothesenknie beispielsweise in der Schwungphase geschehen, in der eine möglichst widerstandsarme Flexion des Kniegelenks gewünscht ist. Die beiden Drosselventile 16, 18, werden geöffnet, sodass ein Fluidfluss zwischen der ersten Zylinderkammer 6 und er zweiten Zylinderkammer 8 möglichst widerstandsarm ermöglicht wird. Dabei kommt es aufgrund der Verringerung des Gesamtvolumens der ersten Zylinderkammer 6 und der zweiten Zylinderkammer 8 zu einem Befüllen des Ausgleichsvolumens 24, was durch den Füllstand 30 angedeutet ist.

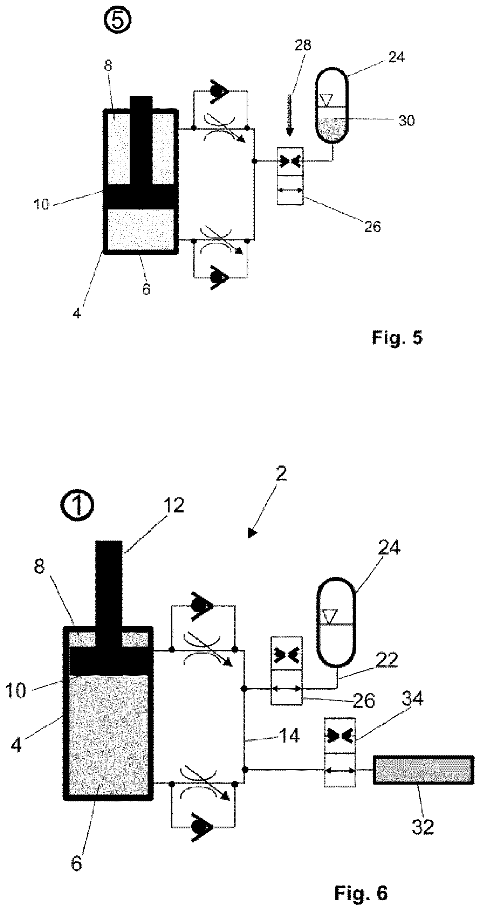

In Figur 5 ist gezeigt, wie in diesem Zustand das erste steuerbare Ventil 26 dem Pfeil 28 entsprechend geschlossen wird. Der Füllstand 30 des Ausgleichsvolumens 24 bleibt unverändert. In diesem Zustand führt ein weiteres Verschieben des Kolbens 10 innerhalb des Zylinders 4 zu einer Veränderung des Gesamtvolumens der ersten Zylinderkammer 6 und der zweiten Zylinderkammer 8, sodass innerhalb des Fluids, beispielsweise des Silikonöls, eine potentielle Energie gespeichert werden kann, die nach dem Wegfall der sie einbringenden Kraft wieder abgegeben wird.

In den Figuren 6 bis 10 ist eine weitere Ausführungsform einer Energiespeichereinrichtung 2 dargestellt. Auch sie verfügt über den Zylinder 4 mit erster Zylinderkammer 6, zweiter Zylinderkammer 8, Kolben 10 und Kolbenstange 12. Das Ausgleichsvolumen 24 ist über das erste steuerbare Ventil 26 schaltbar über die Fluidverbindung 22 mit der Fluidleitung 14 verbunden, in der sich wieder die bereits bekannten Ventile befinden. Zusätzlich zu der Ausgestaltung aus den Figuren 1 bis 5 verfügt die Energiespeichereinrichtung 2 gemäß den Figuren 6 bis 10 über ein Zusatzvolumen 32, das über ein drittes steuerbares Ventil 34 mit der Fluidleitung 14 verbindbar oder von ihr trennbar ist.

In Figur 6 ist sowohl das erste steuerbare Ventil 26 als auch das dritte steuerbare Ventil 34 geöffnet, sodass sowohl das Ausgleichsvolumen 24 als auch das Zusatzvolumen 32 mit der Fluidleitung 14 und damit auch mit der ersten Zylinderkammer 6 und mit der zweiten Zylinderkammer 8 verbunden ist.

Wird eine solche Energiespeichereinrichtung 2 beispielsweise in ein Prothesenknie eingebaut, kann durch die Ausgestaltung das Hinsetzen und insbesondere das spätere Aufstehen des Trägers der orthopädietechnischen Einrichtung, hier also des Prothesenknies, deutlich vereinfacht werden.

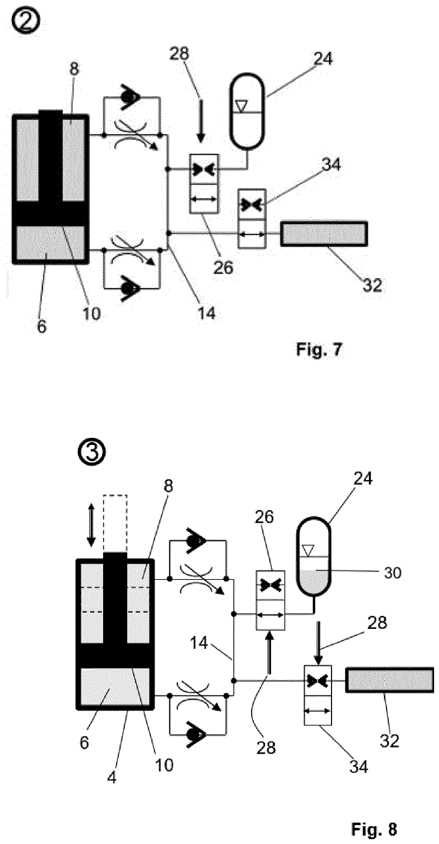

Zum Hinsetzen selbst wird die in Figur 7 gezeigte Schaltanordnung verwendet. Das Schaltventil 26 wird entsprechend es Pfeils 28 geschlossen, sodass das Ausgleichsvolumen 24 von der Fluidleitung 14 abgekoppelt wird. Das dritte steuerbare Ventil 34 bleibt geöffnet. Beim Absenken des Kolbens 10 in die erste Zylinderkammer 6 wird, wie bereits bei den Figuren 1 bis 5 dargelegt, das Gesamtvolumen der ersten Zylinderkammer 6 und der zweiten Zylinderkammer 8 reduziert, woran selbstverständlich auch das noch immer mit der Fluidleitung 14 verbundene Zusatzvolumen 32 nichts ändert. Das dem Arbeitsmedium zur Verfügung stehende Gesamtvolumen wird verkleinert, sodass das Fluid, beispielsweise das Silikonöl, komprimiert wird. In diesem Zustand wird folglich potentielle Energie in der Energiespeichereinrichtung 2 gespeichert.

Nach dem Hinsetzen wir die in Figur 8 gezeigte Schaltanordnung eingestellt. Entsprechend der Pfeile 28 wird das erste steuerbare Ventil 26 geöffnet, sodass das Ausgleichsvolumen 24 mit der Fluidleitung 14 gekoppelt ist. Zudem wird auch das dritte steuerbare Ventil 34 betätigt und in den geschlossenen Zustand gebracht, sodass das Zusatzvolumen 32 vom Rest des Systems abgekoppelt ist. Dabei ist darauf zu achten, dass bevorzugt das dritte steuerbare Ventil 34 vor dem ersten steuerbaren Ventil 26 betätigt wird, um einen vollständigen Druckausgleich auch im Zusatzvolumen 32 zu verhindern. In den beiden Zylinderkammern 6, 8 steht das Arbeitsmedium nach dem Komprimieren während des Hinsetzens, bei dem der Kolben 10 innerhalb des Zylinders 4 abgesenkt wurde, unter einem erhöhten Druck. Wird das erste steuerbare Ventil 26 geöffnet, kann sich dieser Druck entspannen, wobei ein Teil des Fluids in das Ausgleichsvolumen 24 gedrückt wird, was durch den Füllstand 30 dargestellt ist. Werden nun die beiden Drosselventile 16, 18 möglichst weit geöffnet, ist der entgegengesetzte Strömungswiderstand minimal, sodass eine nahezu freie Bewegung des Knies ermöglicht wird. Dies ist insbesondere im sitzenden Zustand gewünscht.

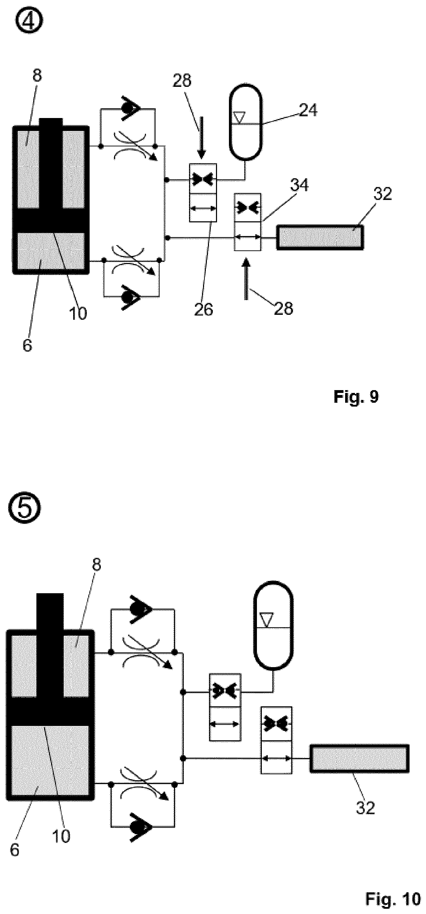

Zum erneuten Aufstehen wird die in Figur 9 gezeigte Schaltanordnung realisiert. Die beiden steuerbaren Ventile 26, 34, werden den Pfeilen 28 entsprechend betätigt. Zunächst wird jedoch der Kolben 10 wieder in die Position gebracht, die einem vollständig flektierten Knie entspricht, das auch beim Hinsetzen erreicht wurde. Anschließend wird das erste steuerbare Ventil 26 betätigt und das Ausgleichsvolumen 24 vom Rest des Fluidsystems getrennt. Anschließend kann das dritte steuerbare Ventil 34 betätigt werden und in den geöffneten Zustand gebracht werden, sodass das Zusatzvolumen 32 wieder mit dem Fluidsystems verbunden ist. In ihm befindet sich noch das unter hohem Druck stehende Fluid, das nun für einen Druckausgleich auch mit den beiden Zylinderkammern 6, 8 sorgt.

Dieser nun erhöhte Druck sorgt für eine nach oben wirkende Kraft auf den Kolben 10, sodass der der Kolben 10 aus dem Zylinder nach oben gedrückt wird. Dies ist in Figur 10 dargestellt. Das in der ersten Zylinderkammer 6, der zweiten Zylinderkammer 8 und dem Zusatzvolumen 32 enthaltene Arbeitsmedium entspannt sich und gibt dabei seine potentielle Energie, die gespeichert ist, ab. Dadurch wird der Kolben 10 nach oben gedrückt und der Träger der orthopädietechnischen Einrichtung, beispielsweise des Prothesenknies, beim Aufstehen unterstützt.

Selbstverständlich sind die Anordnungen auch in andere orthopädietechnische Einrichtungen einbaubar, sodass ein Verschieben des Kolbens 10 innerhalb des Zylinders 4 nicht einer Beugung eines Knies, sondern der Bewegung eines anderen Gelenkes entspricht.

### Bezugszeichenliste

- 2: Energiespeichereinrichtung
- 4: Zylinder
- 6: erste Zylinderkammer
- 8: zweite Zylinderkammer
- 10: Kolben

- 12: Kolbenstange
- 14: Fluidleitung
- 16: erstes Drosselventil
- 18: zweites Drosselventil
- 20: Rückschlagventil

- 22: Fluidverbindung
- 24: Ausgleichsvolumen
- 26: erstes steuerbares Ventil
- 28: Pfeil
- 30: Füllstand

- 32: Zusatzvolumen
- 34: drittes steuerbares Ventil

## Patentansprüche

1. Energiespeichereinrichtung für eine orthopädietechnische Einrichtung, wobei die Energiespeichereinrichtung wenigstens einen Zylinder (4) aufweist, in dem sich
• eine erste Zylinderkammer (6),
• eine zweite Zylinderkammer (8), die mit der ersten Zylinderkammer (6) durch wenigstens eine Fluidleitung (14) fluidtechnisch verbunden ist, und
• ein Kolben (10)
befinden,
wobei der Kolben (10) relativ zu dem Zylinder (4) derart verschiebbar angeordnet ist, dass durch das Verschieben des Kolbens (4) ein Arbeitsmedium, das eine Flüssigkeit ist, durch die wenigstens eine Fluidleitung (14) aus einer Zylinderkammer (6, 8) in die andere Zylinderkammer (8, 6) geleitet wird,
wobei die Energiespeichereinrichtung (2) wenigstens ein Ausgleichsvolumen (24), das mit der Fluidleitung (14) durch eine Fluidverbindung (22) fluidtechnisch verbunden ist, und wenigstens ein erstes steuerbares Ventil (26) aufweist, durch das die Fluidverbindung (22) geöffnet und geschlossen werden kann, **dadurch gekennzeichnet, dass** kein Fluidaustausch zwischen Ausgleichsvolumen und der ersten oder zweiten Zylinderkammer stattfinden kann, wenn das erste steuerbare Ventil geschlossen ist.

2. Orthopädietechnische Einrichtung mit einer Energiespeichereinrichtung (2) nach Anspruch 1.

3. Orthopädietechnische Einrichtung nach Anspruch 2, **dadurch gekennzeichnet, dass** das Arbeitsmedium eine kompressible Flüssigkeit, bevorzugt ein Öl, besonders bevorzugt ein Silikonöl, ist.

4. Orthopädietechnische Einrichtung nach Anspruch 2 oder 3, **dadurch gekennzeichnet, dass** sich in der Fluidleitung (14) wenigstens ein zweites steuerbares Ventil befindet, durch das ein Strömungswiderstand der fluidtechnischen Verbindung vorzugsweise stufenlos einstellbar ist.

5. Orthopädietechnische Einrichtung nach Anspruch 4, **dadurch gekennzeichnet, dass** sich in der Fluidleitung (14) wenigstens zwei zweite steuerbare Ventile befinden, zwischen denen sich die Verbindung zur Fluidverbindung (22) befindet.

6. Orthopädietechnische Einrichtung nach einem der Ansprüche 2-5, **dadurch gekennzeichnet, dass** die Energiespeichereinrichtung (2) wenigstens ein Zusatzvolumen (32) aufweist, das mit der ersten Zylinderkammer (6) und/oder der zweiten Zylinderkammer (8) fluidtechnisch in Verbindung steht, wobei die Energiespeichereinrichtung (2) vorzugsweise ein drittes steuerbares Ventil (34) aufweist, durch das die Verbindung geöffnet und geschlossen werden kann.

7. Orthopädietechnische Einrichtung nach Anspruch 6, **dadurch gekennzeichnet, dass** die Energiespeichereinrichtung (2) mehrere Zusatzvolumina (32) und vorzugsweise mehrere dritte steuerbare Ventile (34) aufweist, durch die die Verbindungen der Zusatzvolumina (32) mit der ersten Zylinderkammer (6) und/oder der zweiten Zylinderkammer (8) vorzugsweise unabhängig voneinander geöffnet und geschlossen werden können.

8. Orthopädietechnische Einrichtung nach Anspruch 7, **dadurch gekennzeichnet, dass** mehrere Zusatzvolumina (32) seriell fluidtechnisch miteinander verbunden sind, und/oder dass mehrere Zusatzvolumnina (32) parallel fluidtechnisch miteinander verbunden sind.

9. Orthopädietechnische Einrichtung nach einem der Ansprüche 2-8, **dadurch gekennzeichnet, dass** die Einrichtung eine elektrische Steuerung aufweist, die eingerichtet ist, die steuerbaren Ventile (26, 34, 16, 18, 20) unabhängig voneinander anzusteuern.

10. Orthopädietechnische Einrichtung nach einem der Ansprüche 2-9, **dadurch gekennzeichnet, dass** der Kolben (10) entlang einer Kreisbahnverschiebbar ist.

11. Orthopädietechnische Einrichtung nach einem der Ansprüche 2-10, **dadurch gekennzeichnet, dass** die Einrichtung eine Knieprothese oder eine Knieorthese ist.

12. Orthopädietechnische Einrichtung nach einem der Ansprüche 2-11, **dadurch gekennzeichnet, dass** ein Durchmesser einer Kolbenstange (12), ein Volumen der ersten Zylinderkammer (6), ein Volumen der zweiten Zylinderkammer (8) und/oder ein Kompressionsmodul des Arbeitsmediums derart gewählt ist, dass sich bei geschlossener Fluidverbindung (22) eine Federkonstante von höchstens 750 N/mm, bevorzugt weniger als 600 N/mm, besonders bevorzugt weniger als 400 N/mm und bevorzugt mehr als 100 N/mm, besonders bevorzugt mehr als 300 N/mm, ergibt.

## Claims

1. An energy storage device (2) for an orthopaedic device, the energy storage device comprising at least one cylinder (4) in which
• a first cylinder chamber (6),
• a second cylinder chamber (8), which is fluidically connected to the first cylinder chamber (6) by means of at least one fluid line (14), and
• a piston (10)
are located,
wherein the piston (10) is arranged such that it can be displaced relative to the cylinder (4) in such a way that the displacement of the piston (4) causes a working medium, which is a liquid, to be directed through the at least one fluid line (14) from one cylinder chamber (6, 8) into the other cylinder chamber (8, 6), wherein the energy storage device (2) comprises at least one compensation volume (24), which is fluidically connected to the fluid line (14) by way of a fluid connection (22), and at least one first controllable valve (26), by way of which the fluid connection (22) can be opened and closed, **characterised in that** no exchange of fluid can occur between the compensation volume and the first or second cylinder chamber when the first controllable valve is closed.

2. An orthopaedic device with an energy storage device (2) according to claim 1.

3. The orthopaedic device according to claim 2, **characterised in that** the working medium is a compressible liquid, preferably an oil, especially preferably a silicon oil.

4. The orthopaedic device according to claim 2 or 3, **characterised in that** there is at least one second controllable valve in the fluid line (14), by means of which a flow resistance of the fluidic connection can preferably be infinitely adjusted.

5. The orthopaedic device according to claim 4, **characterised in that** there are at least two second controllable valves in the fluid line (14), between which the connection with the fluid connection (22) is situated.

6. The orthopaedic device according to one of the claims 2 to 5, **characterised in that** the energy storage device (2) has at least one additional volume (32) that is fluidically connected to the first cylinder chamber (6) and/or the second cylinder chamber (8), wherein the energy storage device (2) preferably comprises a third controllable valve (34), by means of which the connection can be opened and closed.

7. The orthopaedic device according to claim 6, **characterised in that** the energy storage device (2) comprises multiple additional volumes (32) and preferably multiple third controllable valves (34), by means of which the connections between the additional volumes (32) and the first cylinder chamber (6) and/or the second cylinder chamber (8) can be opened and closed preferably independently from each other.

8. The orthopaedic device according to claim 7, **characterised in that** multiple additional volumes (32) are fluidically connected to each other in series and/or that multiple additional volumes (32) are fluidically connected to each other in parallel.

9. The orthopaedic device according to one of the claims 2 to 8, **characterised in that** the device has an electrical control unit that is configured to control the controllable valves (26, 34, 16, 18, 20) independently from each other.

10. The orthopaedic device according to one of the claims 2 to 9, **characterised in that** the piston (10) can be displaced along a circular path.

11. The orthopaedic device according to one of the claims 2 to 10, **characterised in that** the device is a knee prosthesis or knee orthosis.

12. The orthopaedic device according to one of the claims 2 to 11, **characterised in that** a diameter of a piston rod (12), a volume of the first cylinder chamber (6), a volume of the second cylinder chamber (8) and/or a compression module of the working medium is selected in such a way that a closed fluid connection (22) results in a spring constant of at most 750 N/mm, preferably less than 600 N/mm, particularly preferably less than 400 N/mm and preferably more than 100 N/mm, particularly preferably more than 300 N/mm.

## Revendications

1. Dispositif d'accumulation d'énergie (2) pour un dispositif orthopédique, le dispositif d'accumulation d'énergie (2) comprenant au moins un cylindre (4) dans lequel se trouvent
• une première chambre de cylindre (6),
• une deuxième chambre de cylindre (8) qui est en communication fluidique avec la première chambre de cylindre (6) par au moins une conduite de fluide (14), et
• un piston (10),
le piston (10) étant disposé de manière à pouvoir être déplacé par rapport au cylindre (4) de telle sorte que, par le déplacement du piston (4), un fluide de travail, qui est un liquide, est amené d'une chambre de cylindre (6, 8) vers l'autre chambre de cylindre (8, 6) à travers ladite au moins une conduite de fluide (14),
dans lequel
le dispositif d'accumulation d'énergie (2) présente au moins un volume d'équilibrage (24) qui est en communication fluidique avec la conduite de fluide (14) par une liaison de fluide (22), et au moins une première soupape contrôlable (26) permettant d'ouvrir et de fermer la liaison de fluide (22),
**caractérisé en ce que**
aucun échange de fluide entre le volume d'équilibrage et la première ou la deuxième chambre de cylindre ne peut avoir lieu lorsque la première soupape contrôlable est fermée.

2. Dispositif orthopédique comprenant un dispositif d'accumulation d'énergie (2) selon la revendication 1.

3. Dispositif orthopédique selon la revendication 2,
**caractérisé en ce que** le fluide de travail est un liquide compressible, de préférence une huile, de manière particulièrement préférée une huile de silicone.

4. Dispositif orthopédique selon la revendication 2 ou 3,
**caractérisé en ce que** dans la conduite de fluide (14) se trouve au moins une deuxième soupape contrôlable permettant de régler, de préférence en continu, une résistance à l'écoulement de la communication fluidique.

5. Dispositif orthopédique selon la revendication 4,
**caractérisé en ce que** dans la conduite de fluide (14) se trouvent au moins deux deuxièmes soupapes contrôlables entre lesquelles se trouve la communication vers la liaison de fluide (22).

6. Dispositif orthopédique selon l'une des revendications 2 à 5,
**caractérisé en ce que** le dispositif d'accumulation d'énergie (2) présente au moins un volume supplémentaire (32) qui est en communication fluidique avec la première chambre de cylindre (6) et/ou avec la deuxième chambre de cylindre (8), le dispositif d'accumulation d'énergie (2) présentant de préférence une troisième soupape contrôlable (34) qui permet d'ouvrir et de fermer la communication.

7. Dispositif orthopédique selon la revendication 6,
**caractérisé en ce que** le dispositif d'accumulation d'énergie (2) présente plusieurs volumes supplémentaires (32) et de préférence plusieurs troisièmes soupapes contrôlables (34) permettant d'ouvrir et de fermer les communications des volumes supplémentaires (32) avec la première chambre de cylindre (6) et/ou avec la deuxième chambre de cylindre (8), de préférence indépendamment les unes des autres.

8. Dispositif orthopédique selon la revendication 7,
**caractérisé en ce que** plusieurs volumes supplémentaires (32) sont en communication fluidique en série entre eux, et/ou
**en ce que** plusieurs volumes supplémentaires (32) sont en communication fluidique en parallèle entre eux.

9. Dispositif orthopédique selon l'une des revendications 2 à 8,
**caractérisé en ce que** le dispositif comporte une commande électrique qui est conçue pour contrôler les soupapes contrôlables (26, 34, 16, 18, 20) indépendamment les unes des autres.

10. Dispositif orthopédique selon l'une des revendications 2 à 9,
**caractérisé en ce que** le piston (10) peut être déplacé le long d'une trajectoire circulaire.

11. Dispositif orthopédique selon l'une des revendications 2 à 10,
**caractérisé en ce que** le dispositif est une prothèse de genou ou une orthèse de genou.

12. Dispositif orthopédique selon l'une des revendications 2 à 11,
**caractérisé en ce qu'**un diamètre d'une tige de piston (12), un volume de la première chambre de cylindre (6), un volume de la deuxième chambre de cylindre (8) et/ou un module de compression du fluide de travail sont choisis de telle sorte que, lorsque la liaison de fluide (22) est fermée, on obtient une constante de rappel de 750 N/mm au maximum, de préférence de moins de 600 N/mm, de manière particulièrement préférée de moins de 400 N/mm et de préférence de plus de 100 N/mm, de manière particulièrement préférée de plus de 300 N/mm.
